# EUROPEAN PATENT SPECIFICATION

(11) **EP 3 512 931 B1**
(45) Date of publication and mention of the grant of the patent: **05.08.2020**
(21) Application number: 17781571.9
(22) Date of filing: 15.09.2017
(51) Int. Cl.: C12M 1/00, B01F 13/02, B01F 3/04, C12P 21/00, A23K 10/12

(54) **BIOREACTOR FOR AEROBIC HYDROGENOTROPHIC FERMENTATION**
BIOREAKTOR ZUR AEROBEN HYDROGENOTROPHEN GÄRUNG
BIORÉACTEUR POUR LA FERMENTATION HYDROGÉNOTROPHES AÉROBIE

(30) Priority: 15.09.2016 EP 16189030
(43) Date of publication of application: 24.07.2019
(73) Proprietor: KWR Water B.V., 3433 PE Nieuwegein (NL); Avecom NV, 9032 Wondelgem (BE)
(72) Inventor: MATASSA, Silvio, 9000 Gent (BE); VERSTRAETE, Willy Henri, 9032 Wondelgem (BR); VERVAET, Stef, 9000 Gent (BE); WITKAMP, Geert-Jan, 2661 TV Bergschenhoek (NL); HUITING, Hans, 3430 BB Nieuwegein (NL); KREGERSMAN, Bart Jules Henriëtte Maria, 9000 Gent (BE); OESTERHOLT, Franciscus Ignatius Hendrikus Maria, 4142 SJ Leerdam (NL)
(74) Representative: V.O.
(86) International application number: PCT/NL2017/050608
(87) International publication number: WO 2018/052295

(56) References cited:
- EP-A1- 0 418 187
- WO-A1-2013/040012
- JP-A- H03 127 983
- US-A- 3 804 255
- US-A1- 2013 189 763
- US-A1- 2015 031 099
- US-B2- 6 485 003
- MATASSA: "Autotrophic nitrogen assimilation and carbon capture for microbial production by a novel enrichment of hydrogen-oxidizing bacteria", WATER RESEARCH, vol. 101, 26 May 2016 (2016-05-26), pages 137-146, XP029651060, cited in the application

## Description

The invention is in the field of bioreactors dealing with a three-phase system (gas-liquid-solid).

### Introduction

Bioreactors are known, and generally understood, to be vessels which are used to perform biological reactions where organic or inorganic compounds are processed and transformed by microorganisms. Examples are a fermentation vessel to obtain a microbial oil, or a transparent illuminated tank for the production of algae.

Many biological reactions are based on aerobic fermentation. Aerobic fermentation in this context is the biological process of growing microorganisms in a suitable medium, so as to grow the microorganism in the form of biomass suspended in water. Dependent on the type of microorganism, the biomass may be processed and used as such, or it may be further refined to obtain for instance a microbial oil, or it may be used as such. In special cases, genetically modified organisms may be used to obtain specific biological compounds, among which pharmaceuticals.

There are many microorganisms which can be used for aerobic fermentation, and each microorganism requires its specific conditions. Generally, they require an energy source, a carbon source and a nitrogen source, as well as other macro and micronutrients. A specific type of microorganisms uses molecular hydrogen as an energy source. Recently, it has been found that such microorganisms may be useful in converting molecules recovered from biological wastes into protein-rich microbial biomass, generally known as microbial protein. Such microbial protein could be used to help securing the food supply of the growing world population (Matassa et. al., "Can direct conversion of used nitrogen to new feed and protein help feed the world?", Environmental science and technology, 2015, 49, 5247-5254), being more efficient than the conventional agricultural-based food supply. Various microorganisms were shown to be suitable for the efficient conversion of mineral nitrogen into microbial protein by means of aerobic hydrogen gas oxidation (Matassa et. al., "Autotrophic nitrogen assimilation and carbon capture for microbial production by a novel enrichment of hydrogen-oxidizing bacteria", Water Research 101 (2016), 137 - 146). These results were however obtained using a regular completely stirred tank reactor as reaction vessel.

To achieve such conversions on an industrial scale, fermentation reactors which can be operated with molecular hydrogen are required. Molecular hydrogen is a highly volatile and potentially highly explosive gas, which makes its use in industrial processes complicated.

Bioreactors in which hydrogen gas is supplied are known. Such fermentation processes include the use of H₂ and CO₂ or CO, in order to produce methane or alcohol. These are anaerobic fermentations, which do not require (in fact do not tolerate) the presence of molecular oxygen.

Yet, the specific fermentation process for microbial protein production from molecular hydrogen requires the further use of molecular oxygen. The combination of molecular oxygen and molecular hydrogen generally creates a highly explosive mixture. Common bioreactors are not suitable for such use, due to the high explosive risks.

The low solubility of hydrogen in the liquid phase, the high quantities of hydrogen that are required, and the further presence in the reactor of molecular oxygen makes large-scale fermentation of microbial protein from readily available resources challenging. Due to the high quantity of hydrogen which is required for this type of fermentations, hydrogen dissolution is the limiting factor in fermentations to obtain microbial protein, and achieving higher hydrogen dissolution would enhance process efficiency and scale. It is however difficult to achieve a high enough transfer rate of hydrogen from the gaseous phase to the culture medium, especially without using an excess hydrogen which would accumulate into the headspace of or in the closed area surrounding the reactor. Escaping hydrogen gas should be strictly avoided, particularly in reactors where also molecular oxygen is present, due to the explosive risks. In addition, hydrogen gas is expensive, making reactors in which much gas escapes uneconomical. No suitable large-scale bioreactors for this type of aerobic fermentation have yet been described.

WO 2013/040012 describes a bioreactor which applies various gas mixtures of carbon oxides and hydrogen generally in anaerobic setups. The reactor has a straight upright shape and does not allow a specific flow, and also does not apply specific gas inlets. Optionally, the gas feed may be supplemented with oxygen. The reactor in this document suffers from the problem that hydrogen dissolution is not fast enough, so that hydrogen escapes at the top of the reactor which leads to an explosive risk, in particular in embodiments which also use oxygen. In addition, this document is silent on the problem of biomass sedimentation at the hydrogen inlet, and does not mention potential solutions for this problem.

### Description of figures

Figure la-e: various views of the bioreactor.

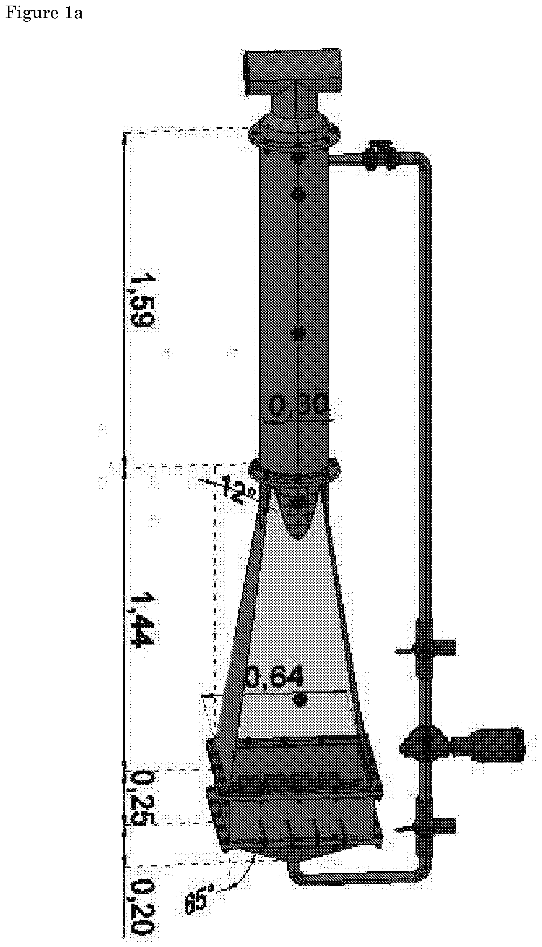

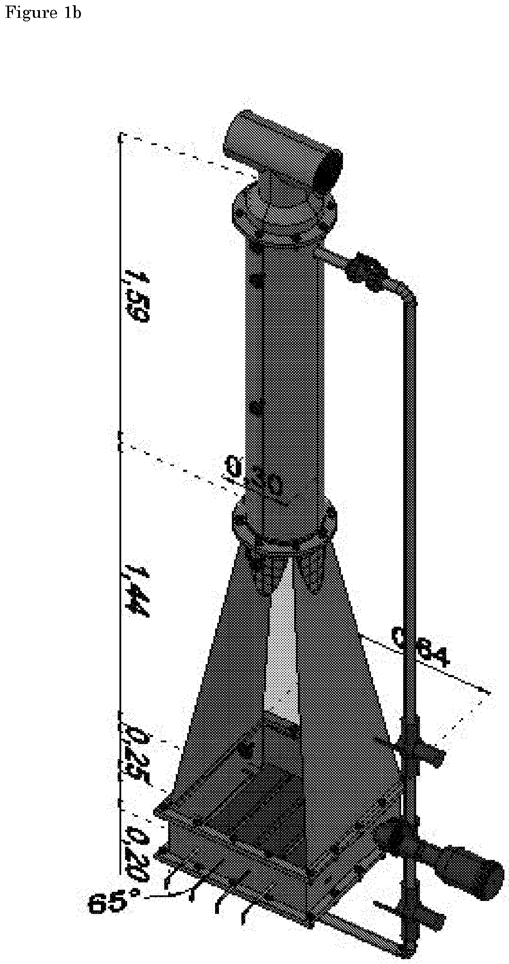

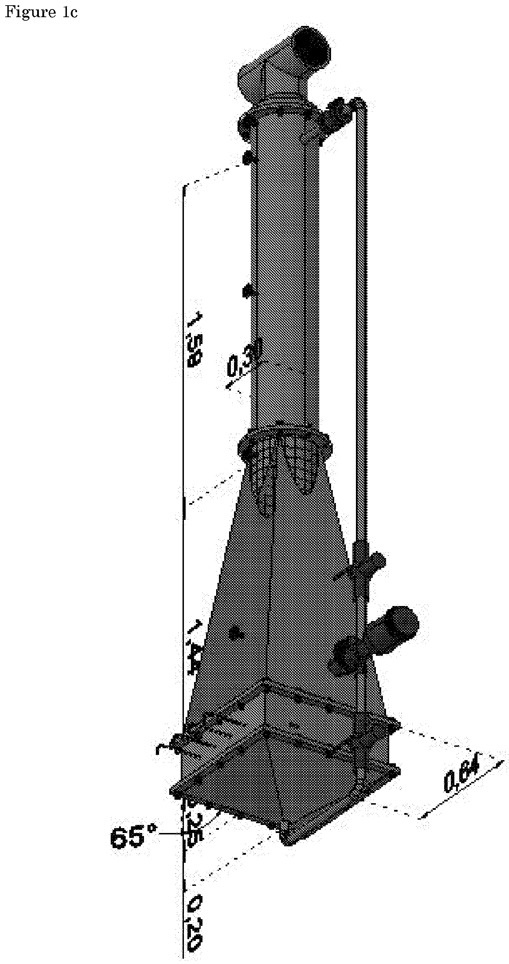

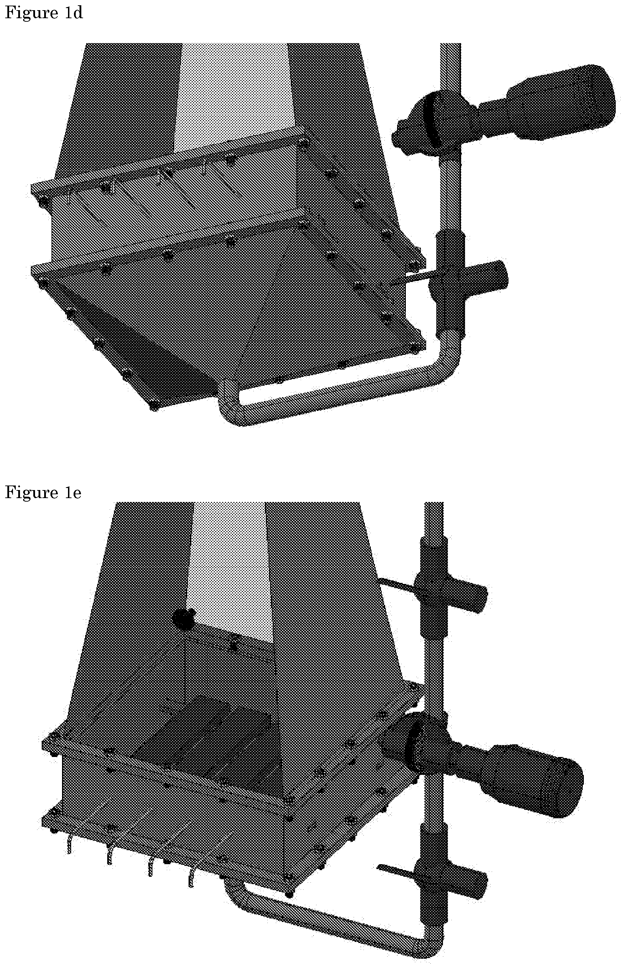

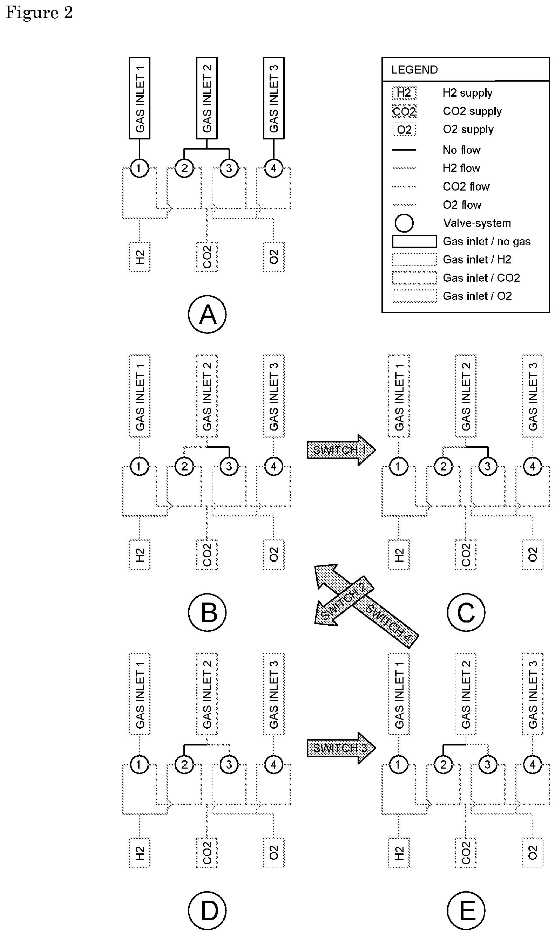

Figure 2: Exemplary gas switch system: A: non-operational mode (valves closed); B: first step of H₂ through first inlet, CO₂ though second inlet, and O₂ through third inlet. Upon switching to the second cycle step, valve 1 changes the feed for the first inlet from H₂ to CO₂, and valve 2 changes the feed for the second inlet from CO₂ to H₂ (Figure C). Upon switching to the third cycle step, valve 1 changes the feed for the first inlet from CO₂ to H₂, and valves 2 and 3 change the feed for the second inlet from H₂ to CO₂ (Figure D). Upon switching a third time, valve 3 changes the feed from the second inlet from CO₂ to O₂ and valve 4 changes the feed from the O₂ to CO₂ (Figure E). A fourth switch returns the system to Figure B.

### Detailed description

The present invention provides a bioreactor for aerobic hydrogenotrophic fermentation, comprising an upper part comprising a top, a bottom part comprising a bottom, an intermediate part comprising a circumferential side face, and a loop adapted to allow flow of a fermentation broth from an exit point in the bottom part to an entry point in the upper part, which bioreactor has an inner diameter (D) and a height (h) defined by the vertical distance between the top and the bottom, and which bioreactor is characterized by
- a pump adapted to provide a circular flow of the fermentation broth from the upper part through the intermediate part to the bottom part of the reactor, and through the loop from the exit point in the bottom part to the entry point in the upper part;
- a first gas inlet, adapted to provide hydrogen bubbles;
- a second gas inlet located at the same height or below the first gas inlet, adapted to provide oxygen;
wherein the intermediate part is defined as the part between the first gas inlet and the entry point in the upper part, and wherein the circumferential side face of the intermediate part comprises a conical portion defined by an inner diameter (D) which varies with the height (h) such that a lower height results in a larger diameter.

It is an advantage of the present reactor that this reactor allows for the large-scale fermentation of hydrogenotrophic microorganisms. This allows for the large-scale production of biomass, in particular single cell protein, from various sources, among which preferably waste products.

The reactor described in the present invention offers several advantages over other types of fermentation systems. The conditions within the fermentation broth ensures that only the microorganisms able to efficiently use hydrogen and oxygen will display appreciable growth. This leads to more efficient conversion of the carbon source (such as for example CO and/or CO₂) and the nitrogen source (such as for example ammonium gas (NH₃) and/or ammonium ions, nitrite ions, nitrate ions, and/or amino acids) into biomass. In this way, the production of biomass will be carried out at higher rates and with increased efficiency. The volumetric productivity of protein can be up to 10 kg biomass/m³ • d, such as 5-10 kg biomasshn^{v} • d, preferably 7.5-10 kg biomass/m³ • d, even more preferably 8.5-10 kg biomass/m³ • d, or preferably even 9-11 kg biomass/m³ • d.

Furthermore, hydrogenotrophic fermentation results in high quality single cell protein, which may have a protein content of up to 70 wt.%. The single cell protein will be referred to as "biomass", which is defined as the whole microorganism cell with all cellular constituents, including protein. The biomass is obtained through fermentation in a fermentation broth which resides inside the reactor. The fermentation broth is a suspension of the biomass in water, wherein the water further comprises feed components, such as an oxygen source, a hydrogen source, a carbon source and a nitrogen source. In a reactor according to the present invention, the hydrogen source is molecular hydrogen (H₂), and the oxygen source is molecular oxygen (O₂). In preferred embodiments, the carbon source is carbon dioxide or carbon monoxide (CO₂ or CO). In further preferred embodiments, the nitrogen source is ammonium gas (NH₃) and/or ammonium ions, nitrite ions, nitrate ions, and/or amino acids.

A further advantage of the present reactor is that a bioreactor according to the present invention is less susceptible to microorganism contamination, because most microorganisms do not tolerate the aggressive environment of the reactor inner contents. Therefore, the present reactor requires less cleaning and can be operated continuously for longer times.

The bioreactor of the invention functions generally as follows. Small hydrogen bubbles emerge from the first gas inlet at the connection between bottom part and intermediate part and may rise upward in a generally vertical motion through the fermentation broth. The circulating fermentation broth, moving from the top of the reactor to the bottom, creates a counterforce, which slows down the vertical upward motion of the hydrogen bubbles. This leads to the advantage that hydrogen bubbles have more contact time with the fermentation broth, which allows the hydrogen bubbles more time to dissolve. In preferred embodiments, the flow is configured such that the hydrogen bubbles have minimal vertical motion, such as for instance less than 1 cm/s, preferably less than 0.5 cm/s, even more preferably less than 0.2 cm/s.

The conical portion of the intermediate part of the reactor has the effect that the downward flow of the fermentation broth at higher parts of the reactor can be relatively fast, while there is slower flow at lower parts of the reactor, where more hydrogen bubbles are not yet dissolved. The downward flow velocity of the broth is lower in the wider reactor section than higher up, but the ratio hydrogen gas/fluid remains the same. The conical portion of the intermediate part functions to prevent coalescence of the bubbles when they reach the top wall of the conical section. At higher parts of the reactor, less but larger bubbles are present, due to dissolution of most of the smaller bubbles, and coalescence of some of the smaller bubbles into larger bubbles. Larger bubbles rise faster, so that a faster flow is required at higher portions of the reactor to slow the vertical motion of those bubbles, and effect dissolution of the larger bubbles.

In combination, these effects make that the reactor of the invention allows for essentially complete dissolution of molecular hydrogen and oxygen. Essentially complete dissolution of hydrogen in this respect means that not more than 7 %, preferably not more than 5 %, more preferably no more than 3 % and most preferably not more than 1 % of the hydrogen supplied to the reactor escapes through the top of the reactor. In an optimized situation, no or essentially no hydrogen gas escapes from the top of the reactor. Thus, formation of an explosive mixture comprising hydrogen and oxygen in the headspace can be avoided. At the same time, the concentration of hydrogen gas in the fermentation broth can be maximized, without the use of excess hydrogen. This is economically advantageous, and bears less explosive risks. The hydrogen concentration in the fermentation broth is generally sufficient to sustain the fermentation of the nitrogen source in the presence of hydrogen, CO₂ or CO, and oxygen into biomass.

It has been found that the effect of the conical portion of the intermediate part on the flow rate of the fermentation broth at various heights, together with the effect of the downward force of the fermentation broth flow which provides for slower rising of the hydrogen bubbles, act synergistically on the dissolution rate of hydrogen. This can be seen from experiments, among which modelling experiments, in which the shape of the reactor is varied and the flow rate optimized. The effect of the conical portion of the intermediate part on the flow rate of the fermentation broth at various heights, and the effect of the downward force of the fermentation broth flow which provides for slower rising of the hydrogen bubbles is that the gas mixture which escapes the top of the reactor is generally not an explosive mixture during operation of the reactor. This is because the quantity of hydrogen which escapes the top of the reactor has been minimized with the present invention, which prevents the buildup of an explosive gas mixture with in particular the also supplied oxygen. At the same time the quantity of hydrogen which dissolves in the reactor has been maximized, providing maximum growth of the microorganisms.

The best results occur in situations where the conical portion varies with height defined by a linear function of the type D = a • h+b wherein a < 0 and b is a positive number, or by an exponential function of the type D = m • n^{h} +c, wherein m and c are positive numbers, and wherein n is between 0 and 1, and wherein the flow rate has been adapted so as to minimize overall vertical motion of the hydrogen bubbles. Such flow rate optimization is dependent on the shape of the reactor, and is preferably such as to result in plug flow throughout the reactor.

Preferably, the circumferential side face has a horizontal cross-sectional surface area which is symmetric at any height, most preferably circular or square. Further preferably, the conical portion of the intermediate part spans the whole intermediate part between the first gas inlet and the entry point which connects the loop to the upper part (i.e., the intermediate part is as a whole conically shaped).

The synergistic effect of the conical section and the counterforce provided by the downward flow of the fermentation broth may be observed by various techniques. Using one or more of these techniques, it can be seen that the exhaust gas stream at the top of the reactor is generally not explosive. In addition, the total quantity of gas which dissolves in the reactor can be inferred.

One option is to measure the total gas exhaust flow from the top of the reactor in a given time, using suitable equipment to determine gas quantity as is known in the art, and determining the composition of the gas exhaust stream, for instance by gas chromatography. This provides direct insight in the quantity and type of gas which escapes, and therefore also in the quantity and type of gas which has dissolved in the reactor (by comparison with the quantity and type of gas which has been supplied through the first and second gas inlet, as the skilled person knows).

Another option is by monitoring the quantity of gas in gaseous state in the fermentation broth. This may be achieved by a detector, which is adapted to display the size of the bubbles in the fermentation broth. Suitable detectors in this regard may function using light scattering, or be based on visualization techniques which are not affected by the fermentation broth inside the reactor. The skilled person can ascertain suitable detector types in this regard. In this way, the bubble size can be determined in time, from which the quantity of gas which escapes may be inferred.

Another way of monitoring the quantity of gas in gaseous state inside the fermentation broth is by determining the density of the fermentation broth. This also provides insight in the dissolution rate of the gases, in particular of hydrogen which dissolves the slowest. Using this technique, the quantity of gas which dissolves may be calculated, from which the quantity of gas which escapes may be inferred.

A further option is to use gas flow meters at the top of the reactor, from which readout the quantity and type of gas which escapes the reactor may also be inferred.

A further option is to determine the rate of microorganisms growth, or the rate of protein formation. As for a particular microorganism, it is known, or can be determined in known ways, the quantities and types of feed required to produce a specific quantity of biomass or protein, the rate of growth also provides insight in the rate of gas consumption, and, by comparison with the quantity of gas introduced, in the quantity and type of gas in the exhaust stream.

Using these techniques (or others, known to the skilled person), it may be seen that the gas exhaust stream at the top of the reactor is generally not explosive. The gas exhaust stream is defined as the gas which escapes from the reactor through the top of the reactor after having been fed to the reactor through the gas inlets (and potentially including any gas that may be liberated by the fermentation process, depending on the type of microorganisms). Thus, the gas in the gas exhaust stream generally comprises less than 4 vol.% hydrogen. Alternatively or additionally, the gas in the gas exhaust stream generally comprises less than 5 vol.% oxygen. In preferred embodiments, the gas in the gas exhaust stream comprises less than 4 vol.% hydrogen and less than 5 vol.% oxygen. In much preferred embodiments, the first gas inlet is configured so as to provide hydrogen bubbles having an average size of 20 - 300 µm.

A further advantage of the present reactor is that it allows for efficient biomass production. This can be observed from the quantity of biomass which is generated per unit time and volume, in comparison to the quantity of H₂ which has been supplied to the reactor. It has been found that the biomass production per unit can be up to 10 kg biomass/m³ • d, such as 5-10 kg biomass/m³ • d, preferably 7.5-10 kg biomass/m³ • d, even more preferably 8.5-10 kg biomass/m³ • d, or even 9-11 kg biomass/m³ • d.

The reactor comprises an upper part comprising a top, a bottom part comprising a bottom, an intermediate part comprising a circumferential side face, and a loop adapted to allow flow of a fermentation broth from an exit point in the bottom part to an entry point in the upper part, which bioreactor has an inner diameter (D) and a height (h) defined by the vertical distance between the top and the bottom. The total height of the reactor is preferably between 2 and 50 m, more preferably between 3 and 30 m, even more preferably between 4 and 15 m. The relatively large height of the reactor has the advantage that the lower parts of the reactor contents operate at relatively high pressure, which ensures faster dissolution of the hydrogen bubbles. In addition, the relatively large height increases the length of the column of fermentation broth through which the hydrogen must pass, further enhancing hydrogen dissolution. Thus, the reactor allows for large-scale production of biomass.

The upper part is connected gas- and liquid tight to the intermediate part, and the intermediate part is connected gas- and liquid tight to the bottom part. This prevents leakage of fermentation broth or undissolved gaseous compounds to the exterior of the reactor. The loop is connected gas- and liquid tight through the entry and exit points to the upper and the bottom parts.

The upper part comprises a top. In preferred embodiments, the top of the reactor is kept open and vented so at all times the risk of accumulation of hydrogen and oxygen at this point is avoided. This makes the reactor inherently safe. In alternative preferred embodiments, the top of the reactor may be closed, such as by a lid. Furthermore, the top may be a hollow void located above the entry point of the loop. The top may be provided with piping which connects the upper part of the reactor with safety equipment, such as sensors, a venting system, or optionally with other reactors. The top part of the reactor preferably has an inner diameter of between 0.05 and 5 m, preferably between 0.1 and 2 m, more preferably between 0.15 and 1 m.

The reactor in general, but preferably the upper part, may comprise a variety of standard safety equipment, which may signal and/or act when the reactor would not function properly. Such safety equipment may include exhaust gas removal equipment, such as fans, air washers or piping which is coupled gas-tight to the inside of the upper part of the reactor. Such piping can be provided with pumps or filters to remove excess gas.

Furthermore, safety equipment can include a variety of detectors, such as in particular gas detectors for detecting hydrogen gas, oxygen gas, CO gas and/or CO₂-gas, which detectors can preferably be coupled to an alarm system which signals the presence of such gaseous compounds at the top of the reactor, or in the space around the reactor. In a most preferred embodiment, such an alarm is automatically coupled to a system which closes at least the gas inlet in question, and preferably all gas inlets, so as to avoid the escape of more gaseous compounds. Further detectors may comprise thermometers, flow rate detectors, and the like.

Safety equipment can also include means for damage control, such as automatic fire extinguishers or strong coolants.

Safety equipment can furthermore include cameras which allow an operator to see the top of the reactor and/or the fermentation broth contained therein.

The upper part includes a gas- and liquid tight connection to the loop through the entry point. The loop allows inflow of the fermentation broth into the reactor, such that the fermentation broth can be circulated over the reactor. Preferably, during operation of the reactor, the height of the surface of the fermentation broth is approximately equal to the height at which the loop connects to the upper part, such as at the same height, or slightly above that height. This allows for the reactor contents flowing in a plug flow, which aids in preventing bubble coalescence and thereby ensures proper dissolution of the gases in the reactor. The point of the entry point where the loop connects to the upper part should be seen as the height where the upper part of the reactor connects to the intermediate part.

The reactor further comprises a bottom part. The height of the first gas inlet is the height at which the bottom part of the reactor connects to the intermediate part. The bottom part has an inner diameter which is wider than the inner diameter of the upper part. The inner diameter of the bottom part is preferably between 0.1 and 6 m, more preferably between 0.2 and 3 m, even more preferably between 0.25 and 1.5 m.

The bottom part comprises a bottom. The bottom may be straight, pyramidal or conically shaped. Preferably, the bottom comprises an outwardly directed protrusion, which leads to the exit point. Further preferably, the bottom is shaped such that the fermentation broth would flow to the exit point under gravity. This can be achieved for instance by (inverted) conical or pyramidal shaped bottoms.

The bottom part comprises an exit point which is connected gas-and liquid tight to the loop. Preferably, the exit point is located in the bottom, such as in a central part of the bottom, preferably in the outwardly directed protrusion. However, the exit point may also be situated on a side of the bottom part. The exit point is situated at a lower height than the first gas inlet. This maximizes hydrogen dissolution, and avoids motion of hydrogen bubbles through the loop to the top part of the reactor.

The bottom part may furthermore be provided with a variety of reactor equipment, such as stirrers, flow monitors or detectors which monitor the presence or absence of a variety of compounds in the fermentation broth.

The reactor furthermore comprises an intermediate part comprising a circumferential side face. The intermediate part is located between the entry point in the upper part and the first gas inlet at the connection of the intermediate part with the bottom part. The intermediate part connects to the bottom part at the height of the first gas inlet, and connects to the upper part at the height of the entry point of the loop. All connections are gas- and liquid tight, so as to provide a vessel in which a fermentation broth can be held without leakage.

The intermediate part is designed such that at least part of the circumferential side face has an inner diameter (D) which is defined as a function of the height (h) such that a lower value for the height results in a larger diameter. The part of the circumferential side face that has a diameter (D) which is defined as a function of the height (h) such that a lower value for the height results in a larger diameter will be referred to as the "conical portion" of the intermediate part, regardless of whether other portions are present, and regardless of the actual shape of the conical portion.

In some embodiments, the intermediate part has an inner diameter such that a lower value for the height results in a larger diameter. In such embodiments, the conical portion spans the complete intermediate part.

In some embodiments, the intermediate part comprises two or more portions, such as an upper and a lower portion, or an upper, middle and lower portion. In such embodiments, one of the portions is the conical portion, whereas the other portion(s) is/are portion(s) in which the inner diameter of the circumferential side face of the reactor is constant with height. Either portion may be the conical portion, and the other portion(s) is/are non-conical.

The conical portion can be a portion in which the part of the circumferential side face which has an inner diameter (D) which varies with height (h) is defined on at least one side by a side-view cross-sectional profile which is linear, concave or convex, with the wider parts of the circumferential side face located at lower height. Preferably, side-view cross-sectional profiles from all sides are linear, concave or convex, with the wider parts of the circumferential side face located at lower height.

Further preferably, the conical portion can be defined from any direction by a side-view cross-sectional profile which is linear, concave or convex. This may be achieved by a side-view which has generally a pyramidal or conical shape, with the wider inner diameters located at lower height.

Both definitions allow for designs wherein the circumferential side face has a horizontal cross-sectional surface area which is asymmetrically shaped. However, it is preferred if the circumferential side face has a horizontal cross-sectional surface area which is symmetric at any height, preferably circular or square. Further preferably, the conical portion preferably has a symmetric side-view, most preferably from each side. This has the advantage that the flow inside the reactor can be more or less uniform.

The conical portion is designed so as to slow the flow of fermentation broth from top to bottom, by increasing the flow diameter and retaining the flow volume constant. Overall, the conical portion is designed so as to provide an overall angle with the vertical of between 1 and 89 °. The overall angle can be determined by interconnecting the top part of the conical portion with the bottom part of the conical portion by a straight line. The angle of this line with the vertical provides the overall angle with the vertical of the conical portion. Preferably, the overall angle of the conical portion is between 2 and 60 °, more preferably between 3 and 45 °, even more preferably between 4 and 30 °, more preferably between 5 and 25 °, most preferably between 8 and 20 °.

The shape of the reactor is not particularly limited. In preferred embodiments, the shape is such so as to provide a more or less uniform flow. Uniform in this context means as much as possible non-turbulent. A non-turbulent flow herein is called plug flow. This may be achieved preferably by a shape of the conical portion which is symmetric, and which further preferably represents a gradual widening of the reactor from top to bottom. Such shapes may be conveniently designed by defining the shape of the reactor by an inner diameter (D) as a mathematical function of the height (h). For instance, the conical portion of the reactor may be defined by a linear function of the type D = a•h+b wherein a < 0 and b is a positive number, or by an exponential function of the type D = m • n^{h} +c, wherein m and c are positive numbers, and wherein n is between 0 and 1. However, the conical portion may also be defined by other mathematical descriptions, or may be random, as long as the conical portion has an inner diameter (D) which is defined as a function of the height (h) such that a lower value for the height results in a larger diameter.

In alternative embodiments, the conical portion is a portion of the intermediate part which is wider at its bottom than at its top, and whose top and bottom are connected by a gradual widening of the intermediate part toward the bottom. This gradual widening may for example be linear, concave or convex, or have other shapes as defined above.

The reactor furthermore comprises a loop. The loop is adapted to allow flow of a fermentation broth from the bottom part to the upper part through the loop. In operation, the flow through the loop is from lower height toward higher height. The height is defined by the vertical distance between the top and the bottom of the reactor, and the top of the reactor is located at higher height than the bottom of the reactor. The loop preferably has a constant diameter over its length, but may have a varying diameter. The loop preferably has a diameter between 0.01 and 1 m, more preferably between 0.02 and 0.75 m, even more preferably between 0.05 and 0.5 m.

The loop may be fitted with additional inlets, such as gas or liquid inlets, or additional outlets, such as outlets for fermentation broth, or safety valves for excess gas. Also, the loop may be fitted with a variety of detectors and safety systems, to monitor the fermentation.

The reactor of the invention further comprises a pump adapted to provide a circular flow of the fermentation broth from the upper part through the intermediate part to the bottom part of the reactor, and through the loop from the exit point in the bottom part to the entry point in the upper part. This pump may be any suitable pump known in the art for the invoking of liquid flow. It should have sufficient power to invoke flow of the full reactor contents. The skilled person is capable of determining what power of the pump is suitable to achieve this, given the total reactor volume and shape.

The reactor further comprises a first gas inlet located at the bottom part of the reactor, which first gas inlet separates the bottom part from the intermediate part, adapted to provide hydrogen bubbles. The hydrogen bubbles preferably have an average size of 20 - 300 µm, preferably 25 - 200 µm, more preferably 30 - 200 µm, at the moment of leaving the gas inlet.

The hydrogen gas bubbles preferably are inserted into the reactor as a continuous stream of hydrogen bubbles, which stream has a horizontal cross-sectional surface area which is at least 15 %, preferably at least 20 %, more preferably at least 30 %, more preferably at least 40 %, more preferably at least 50 %, more preferably at least 60 %, more preferably at least 70 %, and most preferably at least 80 %, of the horizontal cross-sectional surface area of the circumferential side face at the height where the stream emerges from the first gas inlet. This has the advantage that the hydrogen bubbles are introduced into the reactor with sufficient spacing, so as to avoid as much as possible coalescence of the bubbles into larger bubbles. In embodiments in which a relatively large part, such as more than 30 % of the horizontal cross-sectional surface area of the circumferential side face at the height where the stream emerges from the first gas inlet is adapted to provide hydrogen bubbles, is preferred to apply a gas inlet with low flow-block properties, i.e. a gas inlet which allows the fermentation broth to flow through while simultaneously providing hydrogen bubbles. That is, in such embodiments, a tubing system or a fiber net as gas inlet is preferred over a plate or a membrane as gas inlet. This has the effect of allowing the downward flow of the fermentation broth to remain undisturbed, maximizing the residence time of hydrogen in the reactor.

In preferred embodiments, the first gas inlet comprises many sufficiently small holes, distributed evenly over the surface of the first gas inlet, so as to result in a stream of hydrogen gas which is introduced into the reactor as a column of bubbles, in which the bubbles have an average size as defined above.

The first gas inlet (or any other gas inlet) can for instance be a pipe system or sparger plate fitted with holes of 1 - 300 µm, preferably 5 - 200 µm, more preferably 10 - 200 µm, or alternatively, a polymeric or ceramic system with holes of this size. Such a system can for instance also be a mat of hollow fibers, or a system equipped with a membrane, through which hydrogen gas is inserted into the reactor. Other means to create gas bubbles are direct electrolysis in the reactor, by introducing water saturated with gas at higher pressures and/or lower temperatures, by using certain mechanical devices such as nozzles, or by using ultrasound devices.

In some embodiments, the first gas inlet is made up of two or more partial gas inlet systems. The partial gas inlet systems may be connected to a separate gas feed, or they may be connected to the same gas feed. In preferred embodiments, the partial gas inlet systems are identical systems, that do not allow for gas flow from one partial system to the other. This has the advantage separating the first gas inlet, which decreases the volume of hydrogen gas residing in the gas inlet during operation, which increases safety.

The skilled person can readily think of other ways of introducing hydrogen gas in the reactor in the form of small bubbles as defined above, so as to result in a total volume of hydrogen gas in the reactor which is sufficient to sustain the fermentation.

The reactor further is provided with a second gas inlet located at the same height or below the first gas inlet, adapted to provide oxygen. The second gas inlet may provide a constant flow of oxygen bubbles, wherein the oxygen bubbles preferably have a size of 20 - 5000 µm (5 mm), more preferably 25 - 2500 µm, even more preferably 30 - 1000 µm, such as for example 40 - 500 µm. In much preferred embodiments, the oxygen bubbles have the similar size as the hydrogen bubbles, such as 20 - 300 µm, preferably 25 - 200 µm, more preferably 30 - 200 µm, at the moment of leaving the second gas inlet.

The second gas inlet may be a similar system as defined above for the first gas inlet. In addition, many means for supplementing oxygen in the form of bubbles to a reactor are known, which may also be applicable.

The first and second gas inlet are preferably connected gas-tight to an oxygen gas feed and a hydrogen gas feed. The gas feed may be bottles with the gas in question, which are commercially available. Alternatively, hydrogen and oxygen gas may be generated on site by electrolysis of water, as is known in the art, and introduced into the reactor through the appropriate gas inlet.

Preferably, the reactor is equipped with one or more additional inlets, adapted to introduce a carbon source, and/or a nitrogen source into the reactor.

The one or more additional inlets may comprise one or more additional gas inlets adapted to provide as carbon source gaseous carbon dioxide and/or gaseous carbon monoxide, and/or as nitrogen source gaseous ammonia, or alternatively, ammonium ions, nitrite ions, nitrate ions, and/or amino acids, which are preferably supplied in the form of a solution in water.

The one or more additional gas inlets may have any suitable shape to introduce gas into a liquid system. Gas inlets which provide gas as small bubbles, such as bubbles with a size of 20 µm - 5 mm, are preferred, preferably 25 - 2500 µm, even more preferably 30 - 1000 µm, such as for example 40 - 500 µm. In other preferred embodiments, the additional gas inlets provide bubbles of similar size as the hydrogen bubbles, such as 20 - 300 µm, preferably 25 - 200 µm, more preferably 30 - 200 µm, at the moment of leaving the gas inlet.

Additional gas inlets are preferably located at the bottom part of the reactor. In a preferred embodiment, the first gas inlet and the additional gas inlets are of the same type, so as to provide uniform mixing of the gases with the fermentation broth

The additional gas inlets, are preferably connected to a gas reservoir, from which gas can be fed through the gas inlets to the reactor. The required gases may be fed for instance from gas bottles, which are commercially available. The one or more additional inlets may also comprise one or more liquid inlets. The liquid inlet(s) may be located at the same or at different parts of the reactor. Liquid inlets are also preferably connected to a reservoir or other system from which liquids can be fed to the reactor. In a preferred embodiment, the reactor comprises a liquid inlet providing as a nitrogen source an aqueous solution comprising ammonium ions, nitrite ions, nitrate ions, and/or amino acids. Furthermore, the one or more liquid inlets may comprise a water inlet.

The liquid nitrogen source comprising ammonium ions, nitrite ions, nitrate ions, and/or amino acids may be aqueous solutions obtained by mixing commercially obtained ingredients.

Preferably however, the aqueous solution comprising ammonium ions, nitrite ions, nitrate ions, and/or amino acids has been obtained from waste or sewage water. Such water is rich in soluble nitrogen compounds, suitable for the fermentation of hydrogen oxidizing bacteria. After anaerobic digestion of the waste or sewage water, which is known in the art, the aqueous solution comprises as nitrogen source ammonium ions and/or amino acids, which is a highly preferred solution for use in the present reactor. The recovery and the use of these compounds as source of nitrogen in the production of microbial protein offers a way to increase the efficiency of the anthropogenic nitrogen cycle created by the conventional feed and food chain.

When using these nitrogen compounds as feed to produce biomass directly from microorganisms, the conversion of nitrogen compounds to biomass is more efficient than when the same compounds would be used as fertilizer, and plants (or animals) would be used to convert the compounds to biomass. Biomass can be produced more directly, and therefore more efficiently, from wastewater, and the presently invented bioreactor allows for large-scale fermentation of microorganisms which allow for this conversion.

The reactor preferably also comprises at least one liquid outlet. The outlet can be used to remove the fermentation broth from the reactor during or after fermentation, for instance with the purpose of isolating biomass from the fermentation broth. The liquid outlet may be located at any point of the reactor, but preferably, it is located in the bottom part of the reactor.

Thus, in preferred embodiments, the reactor comprises a third gas inlet, adapted to provide carbon monoxide, carbon dioxide, or a mixture of carbon monoxide and carbon dioxide.

In further preferred embodiments, the reactor comprises
(a) a fourth gas inlet, adapted to provide ammonia gas, and/or
(b) a liquid inlet, adapted to provide a solution comprising ammonium ions, nitrite ions, nitrate ions, and/or amino acids.

All inlets may be dynamically controlled, so as to respond to increasing or decreasing concentration of a reaction components, to keep the various components in a specific ratio, defined below.

The reactor preferably also comprises a variety of regulators for process parameters, such as means to control the temperature, the pressure, the flow rate, as well as other conventional process parameters. The reactor may furthermore comprise various other elements, known to be practical in the operation of fermentation reactors. As an example, it is advantageous to include means for cooling the reactor, preferably in conjunction with one or more heat pumps to recycle the heat.

As mentioned above, the reactor may, at suitable locations not limited to those defined above, further be equipped with suitable control equipment, safety equipment, and the like, so as to allow safe and efficient operation of the reactor while performing a fermentation.

The reactor may be configured to function batch-wise, semi-continuous or continuous. For allowing (semi) continuous operation, it may be convenient to include further gas or liquid inlets at suitable locations, so as to (semi) continuously add and remove reaction mixture or reaction mixture components during the fermentation.

The reactor volume preferably allows for pilot scale and/or industrial scale fermentations. Thus, the reactor preferably has a volume of at least 5 dm³, more preferably at least 10 dm³, even more preferably at least 20 dm³, even more preferably at least 50 dm³, even more preferably at least 100 dm³, even more preferably at least 200 dm³, even more preferably at least 250 dm:³, even more preferably at least 500 dm³, even more preferably at least 1000 dm³, even more preferably at least 2000 dm³, even more preferably at least 5000 dm³. There is no particular limit to the upper value, but preferably this is for instance 10000, 20000 or 30000 dm³. The large volume increases the pressure in the bottom part, which accelerates hydrogen dissolution.

In some embodiments, the bioreactor is adapted to withstand an increased inner pressure, such as a pressure of 1.1 - 15 bar, preferably 2 - 10 bar. This results in even further increased hydrogen dissolution. In such embodiments, appropriate gas and liquid locks must be in place, as is known in the art.

The reactor can be made from any suitable material, such as ceramics, glass, polymeric materials or metal. Preferred materials are stainless steel, polypropylene, PVC, and other robust materials capable of withstanding the forces associated with circulating fermentation broth while not affecting the reaction, and not leaking into the broth compounds which may affect biomass quality. Combinations of material are also possible, such as stainless steel, provided with windows to allow visualization of the reactor's content.

The reactor is preferably configured for fermentation of hydrogen-oxidizing bacteria. Hydrogen oxidizing bacteria are defined as bacteria which are capable of converting molecular hydrogen into biomass. Hydrogen oxidizing bacteria are aerobic microorganisms, which have the ability to fix a carbon source, such as carbon monoxide or carbon dioxide, into cellular material, using hydrogen as electron donor and oxygen as electron acceptor. For this conversion, further nutrients may be required, such as a nitrogen source and other macronutrients such as phosphorus, potassium etc.. In preferred embodiments, micronutrients such as metals, salts and the like may also be used.

Hydrogen oxidizing bacteria include for instance knallgas bacteria, such as known to exist in the genus of *Ralstonia, Proteobacteria, Aquificales, Actinobacteria, Firmicutes. Sulfuricurvum, Alcaligenes, Cupriavidus, Thermomonas, Chryseobacterium, Ancylobacter, Hydrogenophaga, Xanthobacter, Bdellovibrio, Falvobacteria, Sphingobacteria, Ideonella* or *Streptomyces,* preferably *Ralstonia, Cupriavidus, Alcaligenes or Sulfuricurvum.* Other known knallgas bacteria may also be used.

Preferred hydrogen-oxidizing bacteria are *Cupriavidus necator, Ralstonia eutropha, Alcaligenes eutrophus and Sulfuricurvum kuijense.*

It has been found that in a bioreactor for aerobic hydrogenotrophic fermentation, biomass has the tendency to have highest growth at the gas inlet which provides hydrogen. This may cause the problem of clogging the gas inlet which provides hydrogen by biomass sedimentation at the hydrogen gas inlet. A further problem is that such sedimentation prevents the formation of small bubbles. Essentially no biomass sedimentation is observed at the gas inlet which provides oxygen. It is a further advantage of the present invention that clogging of the hydrogen gas inlet can be avoided using one or a combination of the following setups.

This may be achieved through a bioreactor comprising at least two inlets as defined above, which inlets are adapted to provide either gas bubbles of 20-300 µm, or liquid, such that each inlet can switchably provide as hydrogen source hydrogen gas, such that hydrogen gas is supplied to the reactor from a different inlet at a different time, and such that hydrogen gas is not supplied simultaneously with oxygen gas through the same inlet.

This is also possible with a reactor comprising inlets selected from at least two gas inlets and optionally at least one liquid inlet, wherein each gas inlet is adapted to provide gas bubbles of 20-300 µm, such that each inlet can switchably provide as hydrogen source hydrogen gas, as oxygen source oxygen gas, as carbon source carbon monoxide or carbon dioxide, and as nitrogen source ammonia gas, ammonium ions, nitrite ions, nitrate ions, and/or amino acids.

In such embodiments, the hydrogen is alternatingly provided through different inlets, and hydrogen is not provided simultaneously with oxygen gas through the same inlet. This is further preferably achieved through a cycle in which at no time oxygen is being provided through an inlet where in the preceding step hydrogen has been provided, and in which cycle at no time hydrogen is provided through an inlet where in the preceding step oxygen has been supplied. Thus, before and after providing hydrogen through an inlet, that inlet is used to provide a liquid or a gas different from hydrogen or oxygen, and before and after providing oxygen through an inlet, that inlet is used to provide a liquid or a gas different from hydrogen or oxygen.

In preferred embodiments, the liquid is water, such as hot water defined elsewhere. In other preferred embodiments, the liquid may be a liquid feed, such as a solution comprising ammonium ions, nitrite ions, nitrate ions and/or amino acids.

In a first, much preferred embodiment, called a "gas switch", both the first gas inlet and the second gas inlet can be adapted to provide either hydrogen bubbles or oxygen bubbles as defined above, such that the second gas inlet provides oxygen bubbles when the first gas inlet provides hydrogen bubbles, and the second gas inlet provides hydrogen bubbles when the first gas inlet provides oxygen bubbles, characterized in that the first and second gas inlet are switchably connected to an oxygen gas feed and a hydrogen gas feed, such that the first gas inlet and the second gas inlet alternatingly provide hydrogen gas bubbles and oxygen gas bubbles.

A switchable connection, in this regard, is a connection which allows for alternating supplementation with two (or more) different gases, or of one or more gases, and of one or more liquids. This allows for instance for hydrogen supplementation and oxygen supplementation, essentially without delay in gas supplementation and essentially without supplementation of a hydrogen/oxygen gas mixture. A switchable connection may be made by application of appropriate tubes and switches using appropriate valves, as is known in the art.

In this setup, the reactor is operated in a two phase alternating cycle, in which a first phase is alternated with a second phase. In the first phase, hydrogen bubbles are supplied through the first gas inlet and oxygen bubbles are supplied through the second gas inlet. In the second phase, oxygen bubbles are supplied through the first gas inlet and hydrogen bubbles are supplied through the second gas inlet. Oxygen gas and hydrogen gas are not supplied through the same gas inlet, in order to avoid explosive risk.

In this setup, it is preferred that the first and the second gas inlet are located at about equal height in the reactor, in which case the first and second gas inlet preferably have an identical setup. However, in embodiments where the first and second gas inlet are located in the reactor at different height, the first gas inlet is the gas inlet located the highest, irrespective of whether the gas inlet provides hydrogen or oxygen at a given point in the operating cycle.

It is further preferred that the first and second gas inlet are identical, at least on technical aspects such as position height in the reactor, quantity, size and distribution of holes, and gas throughput speed. This ensures that the reactor can be operated constantly, without changes to the position of the steady state equilibrium between biomass and feed components. In much preferred embodiments, the first and second gas inlet are adapted to alternatingly provide hydrogen and oxygen, in a system wherein the first and second gas inlet have an equal number of equally spaced holes, positioned at the same height in the reactor. The holes of the first and second gas inlet in this embodiment are for example 1 - 300 µm, preferably 5 - 200 µm, more preferably 10 - 200 µm.

This may be achieved for instance using an interwoven net of fibers or tubing, or a pipe system, or a polymeric or ceramic system, wherein half of the volume of the gas feed system is switchably connected to both the hydrogen feed and the oxygen feed, and the other half of the volume of the gas feed system is also switchably connected to both the hydrogen feed and the oxygen feed, and wherein no mixing of hydrogen and oxygen occurs.

An example is an interwoven network of fibers, wherein the fibers in one direction are provided with holes of a particular shape, size and distribution, which fibers together function as the first gas inlet. The fibers of the interwoven network which are positioned perpendicular to the fibers which make up the first gas inlet are provided with holes of identical shape, size and distribution as the holes of the first gas inlet, and function as the second gas inlet. By switchably connecting the first gas inlet and the second gas inlet to the hydrogen feed and the oxygen feed, the first gas inlet and the second gas inlet can alternatingly provide hydrogen gas bubbles and oxygen gas bubbles which are identical in size, and originate at the same height in the reactor, so as to assure a constant feed composition during operation of the reactor. In one phase of the operating cycle, hydrogen is provided to the reactor through the first gas inlet and oxygen is provided through the second gas inlet. At the next step of the operating cycle, hydrogen is supplied through the second gas inlet, and oxygen is supplied through the first gas inlet, essentially without delay during switching, and essentially without the mixing of hydrogen and oxygen in the first and second gas inlet. The same effect may be achieved using a system of crossed pipes or tubing, or with a polymeric or ceramic system.

This alternating setup has the effect that biomass which sedimented at the first gas inlet at a point in time in the cycle when the first gas inlet supplied hydrogen, is removed from the first gas inlet at points in the cycle in which the first gas inlet provides oxygen. The same is true for the second gas inlet. Thus, biomass which sediments on a gas inlet during hydrogen supplementation is removed from that gas inlet during oxygen supplementation. Using this setup, biomass sedimentation can be avoided by operating the reactor alternatingly between the two setups. The first and second phase of the operating cycle are preferably of approximately equal duration.

In an alternative gas-switch setup to prevent biomass sedimentation, the reactor comprises at least three gas inlets, which are preferably identical as described above. In preferred embodiments, the three gas inlets are the first gas inlet, the second gas inlet, and the third gas inlet. The three gas inlets are switchably connected to the hydrogen feed, the oxygen feed and the carbon source, such as carbon monoxide or carbon dioxide. In this setup, it is also possible to avoid biomass sedimentation as described above, but this setup has the additional advantage that a cycle may be defined such that at no time the mixing of hydrogen and oxygen occurs, by using a cycle which prevents oxygen being supplied through an inlet where in the preceding step hydrogen has been supplied, and which prevents hydrogen being supplied through an inlet where in the preceding step oxygen has been supplied. This may be achieved for instance by providing through an inlet which in the preceding cycle step provided hydrogen or oxygen, a gaseous carbon- or nitrogen source. In the subsequent cycle step, that same inlet may then again provide hydrogen or oxygen. Such a cycle allows for instance to provide gaseous carbon or nitrogen source between hydrogen and oxygen supplementation, thereby preventing the mixing of hydrogen and oxygen in the feed system of a gas inlet.

In much preferred embodiments, each gas inlet at some point in the cycle provides carbon monoxide. This has the further advantage that the gas inlets are disinfected by the carbon monoxide, due to a sudden pH-drop.

In a second embodiment, called the "liquid wash", clogging by biomass of the inlet which provides hydrogen can be avoided by flushing the inlet which provides hydrogen with a liquid, which liquid is preferably water, and which liquid is preferably hot. In an example of this embodiment, the first gas inlet can be switchably connected to a hydrogen feed and a liquid feed, similar to the above-described setup which alternatingly provides hydrogen and oxygen. This allows the reactor to be operated in a two-phase alternating cycle, in which a first phase is alternated with a second phase, wherein in the first phase hydrogen is provided through the gas inlet, and in the second phase, the liquid is provided through the gas inlet. In this embodiment, the gas inlet must not only be adapted to provide hydrogen, but must also be adapted to provide the liquid.

The liquid is preferably water, and the liquid is preferably hot, such as for example at a temperature of 40 - 80 °C, preferably 50 - 70 °C. Preferably, the temperature of the liquid is higher than the temperature at which the reactor is operated. In preferred embodiments, the heat of the liquid is derived at least partially from a heat pump, by exchange with the heat taken up in cooling water used to cool the reactor. When using hot liquid at such temperatures, the heat of the liquid when exiting the inlet is high, which effectively removes biomass. However, the heat of the liquid is almost immediately reduced by dilution with the contents of the reactor, so that the rest of the biomass in the reactor is not or barely affected by the hot liquid. This has the advantage of efficiently removing biomass from the hydrogen inlet, without appreciably affecting the rest of the biomass in the reactor.

In further preferred embodiments, the liquid may comprise hydrogen peroxide or acid, in a quantity low enough to not affect the fermentation.

In further preferred embodiment, the liquid may be a liquid feed, such as a solution comprising ammonium ions, nitrite ions, nitrate ions and/or amino acids. This has the additional advantage that the liquid is simultaneously used for providing the nitrogen source, as well as for the removal of sedimented biomass. This has the further advantage of a less complicated construction, which increases the technical reliability of the reactor.

In preferred setups of this embodiment, supplementation of the liquid occurs simultaneously with removal of some fermentation broth from the reactor, such as through the liquid outlet. It is preferred if the volume of liquid supplied through the inlet is approximately equal to the volume of fermentation broth removed through the liquid outlet.

Of course, it is possible to apply a combination of a liquid wash setup with a gas-switch setup. In such cases, it is preferred if both the first gas inlet and the second gas inlet, and potentially also other gas inlets, are adapted to provide either a liquid or a gas. In this embodiment, the reactor may be operated in a three-phase cycle, in which an inlet may provide in any order hydrogen, oxygen and liquid, although there is at no time supplied a combination of hydrogen and oxygen.

In much preferred embodiments, the reactor comprises a first, a second, a third and a fourth inlet, wherein each inlet is adapted to provide gas bubbles or liquid. The reactor may then be operated in a four-stage cycle, wherein each step is preferably of approximately equal duration, using a cycle which prevents oxygen being supplied through an inlet where in the preceding step hydrogen has been supplied, and which prevents hydrogen being supplied through an inlet where in the preceding step oxygen has been supplied. This may for example be achieved using the following cycle steps
1) the first inlet provides hydrogen gas, the second inlet provides carbon source, the third inlet provides oxygen gas, and the fourth inlet provides nitrogen source;
2) the first inlet provides carbon source, the second inlet provides oxygen gas, the third inlet provides nitrogen source, and the fourth inlet provides hydrogen gas;
3) the first inlet provides oxygen gas, the second inlet provides nitrogen source, the third inlet provides hydrogen gas, and the fourth inlet provides carbon source;
4) the first inlet provides nitrogen source, the second inlet provides hydrogen gas, the third inlet provides carbon source, and the fourth inlet provides oxygen gas.

The skilled person readily understands the cycle steps may be varied so as to achieve the same goal of avoiding biomass sedimentation as well as allowing operation at increased safety and/or reliability, by applications of gas-switch and/or liquid wash setups.

The switchable connection between the gas or liquid feed systems and the inlets may be achieved by one or more valves, mounted in the feed system, such as in the piping or tubing. The valve connects a feed system, such as the system which feeds hydrogen to an inlet, with another feed system, such as the system which provides oxygen to an inlet. Also more than two feed systems may be connected to a single valve. Appropriate valves for creating a switchable feed system are known in the art. Valves may connect two or more gas feed systems to two or more inlets, but may also be used to create a switchable connection between a gas feed system and a liquid feed system, and two inlets adapted to provide either liquid or gas.

The invention furthermore pertains to a method for the production of biomass, comprising
- providing a bioreactor as defined above with sufficient water, sufficient carbon source, sufficient oxygen source, sufficient nitrogen source, and sufficient nutrients to sustain a fermentation, and with an inoculum comprising hydrogen oxidizing bacteria, and providing hydrogen bubbles having an average size of 20-300 µm, preferably 25 - 200 µm, more preferably 30 - 200 µm, to obtain a fermentation broth;
- operating the pump to circulate the fermentation broth from the upper part through the intermediate part to the bottom part of the reactor, and through the loop from the bottom part to the upper part, such that the hydrogen bubbles essentially completely dissolve in the fermentation broth;
- continuing fermentation until an extractable quantity of biomass has generated;
- isolating biomass from the fermentation broth.

The skilled person is capable of determining what quantities of water, carbon source, oxygen source, nitrogen source, and nutrients are sufficient to sustain a fermentation. This depends among others on the stage of the fermentation, as well as on the exact microorganism used, and can readily be determined by trial runs. Suitable quantities for steady-state fermentation of specific microorganisms are given in Matassa et.al., "resource recovery from used water: the manufacturing abilities of hydrogen-oxidizing bacteria", Water Research 68 (2015), 467-478.

In a preferred embodiment, the carbon source is gaseous carbon dioxide or carbon monoxide, the oxygen source is gaseous oxygen, and the nitrogen source is either gaseous ammonia or a solution comprising ammonium ions.

The inoculum comprising the microorganism can be provided to the reactor at any suitable time. Preferably, the inoculum is provided when the mixture inside the reactor has a composition suitable to sustain the fermentation.

In preferred embodiments to sustain fermentation, the volumetric ratio between hydrogen and oxygen is between 0.5 : 1 and 10 : 1, preferably between 1 : 1 and 5 : 1. Further preferably, the ratio between oxygen and carbon dioxide or carbon monoxide is between 1 : 0.1 and 1 : 1.5, preferably between 1 : 0.5 and 1 : 1. Further preferably, the quantity of nitrogen in the nitrogen source is 0.005 - 0.1 kg N per m³ H₂, preferably 0.01 - 0.05 kg N per m³ H₂. Further preferably, the volumetric ratio between hydrogen an CO or CO₂ is between 2 : 1 and 10 : 1, preferably between 4 : 1 and 6 : 1, most preferably between 5 : 1 and 5.5 : 1.

The reactor is preferably operated at a temperature which provides appreciable growth of the microorganism in question. Thus, the operating temperature of the reactor is generally 5 - 50 °C, preferably 10 - 45 °C, more preferably 15 - 40 °C.

In preferred embodiments, the reactor is operated under pressure, such as a pressure of 1.1 - 15 bar, preferably 2 - 10 bar, which increases hydrogen dissolution even further.

For the purpose of clarity and a concise description features are described herein as part of the same or separate embodiments, however, it will be appreciated that the scope of the invention may include embodiments having combinations of all or some of the features described.

## Claims

1. A bioreactor for aerobic hydrogenotrophic fermentation, comprising an upper part comprising a top, a bottom part comprising a bottom, an intermediate part comprising a circumferential side face, and a loop adapted to allow flow of a fermentation broth from an exit point in the bottom part to an entry point in the upper part, which bioreactor has an inner diameter (D) and a height (h) defined by the vertical distance between the top and the bottom, and which bioreactor is **characterized by**
- a pump adapted to provide a circular flow of the fermentation broth from the upper part through the intermediate part to the bottom part of the reactor, and through the loop from the exit point in the bottom part to the entry point in the upper part;
- a first gas inlet, adapted to provide hydrogen bubbles;
- a second gas inlet located at the same height or below the first gas inlet, adapted to provide oxygen;
wherein the intermediate part is defined as the part between the first gas inlet and the entry point in the upper part, and wherein the circumferential side face of the intermediate part comprises a conical portion defined by an inner diameter (D) which varies with the height (h) such that a lower height results in a larger diameter.

2. A bioreactor according to claim 1, wherein the hydrogen bubbles have an average size of 20 - 300 µm.

3. A bioreactor according to claim 1 or 2, wherein the first gas inlet is configured to provide a continuous stream of hydrogen bubbles, which stream has a horizontal cross-sectional surface area which is at least 15 % of the horizontal cross-sectional surface area of the circumferential side face at the height where the stream emerges from the first gas inlet.

4. A bioreactor according to any of claims 1 - 3, wherein the circumferential side face of the conical portion of the intermediate part is defined on at least one side by a side-view cross-sectional profile which is linear, concave or convex.

5. A bioreactor according to any of claims 1 - 4, wherein the inner diameter of the conical portion varies with height defined by a linear function of the type D = a • h+b wherein a < 0 and b is a positive number, or by an exponential function of the type D = m • n^{h} +c, wherein m and c are positive numbers, and wherein n is between 0 and 1.

6. A bioreactor according to any of claims 1 - 5, further comprising
(a) one or more additional inlets, adapted to introduce a carbon source, and/or a nitrogen source into the reactor; and/or
(b) a third gas inlet adapted to provide a carbon source, wherein the carbon source is carbon monoxide, carbon dioxide, or a mixture of carbon monoxide and carbon dioxide; and/or
(c) a fourth gas inlet, adapted to provide as nitrogen source, ammonia gas, and/or
(d) a liquid inlet, adapted to provide as nitrogen source a solution comprising ammonium ions, nitrite ions, nitrate ions, and/or amino acids.

7. A bioreactor according to any of claims 1 - 6, configured for fermentation of hydrogen oxidizing bacteria.

8. A bioreactor according to any of claims 1 - 7, wherein the circumferential side face has a horizontal cross-sectional surface area which is symmetric at any height.

9. A bioreactor according to any of claims 1 - 8 comprising at least two inlets, which inlets are adapted to provide either gas bubbles of 20-300 µm, or liquid, such that the hydrogen bubbles are alternatingly provided from a different inlet.

10. A bioreactor according to any of claims 1 - 9, wherein both the first gas inlet and the second gas inlet are adapted to provide either hydrogen bubbles or oxygen bubbles, such that the second gas inlet provides oxygen bubbles when the first gas inlet provides hydrogen bubbles, and the second gas inlet provides hydrogen bubbles when the first gas inlet provides oxygen bubbles, wherein the first and second gas inlet are switchably connected to an oxygen gas feed and a hydrogen gas feed, such that the first gas inlet and the second gas inlet alternatingly provide hydrogen gas bubbles and oxygen gas bubbles.

11. A bioreactor according to any of claims 1 - 10, wherein the first gas inlet is adapted to provide either hydrogen bubbles or a liquid, wherein the first gas inlet is switchably connected to a hydrogen gas feed and a liquid feed, such that the first gas inlet alternatingly provides hydrogen gas bubbles and liquid, wherein preferably the liquid is water, which water optionally comprises hydrogen peroxide and/or acid, which water preferably has a temperature of 40-80 °C, or wherein the liquid is an aqueous solution comprising ammonium ions, nitrite ions, nitrate ions and/or amino acids.

12. A method for the production of biomass, comprising
- providing a bioreactor according to any of claims 1 - 11 with sufficient water, sufficient carbon source, sufficient oxygen source, sufficient nitrogen source, and sufficient nutrients to sustain a fermentation, and with an inoculum comprising hydrogen oxidizing bacteria, and providing hydrogen bubbles having an average size of 20-300 µm, to obtain a fermentation broth;
- operating the pump to circulate the fermentation broth from the upper part through the intermediate part to the bottom part of the reactor, and through the loop from the bottom part to the upper part, such that the hydrogen bubbles essentially completely dissolve in the fermentation broth;
- continuing fermentation until an extractable quantity of biomass has generated;
- isolating protein from the fermentation broth.

13. A method according to claim 12, wherein the carbon source is gaseous carbon dioxide or carbon monoxide, the oxygen source is gaseous oxygen, and the nitrogen source is either gaseous ammonia or a solution comprising ammonium ions, nitrite ions, nitrate ions, and/or amino acids, wherein preferably, the volumetric ratio between hydrogen and oxygen is between 0.5 : 1 and 10 : 1, preferably between 1 : 1 and 5 : 1, and wherein the ratio between oxygen and carbon dioxide is between 1 : 0.1 and 1 : 1.5, preferably between 1 : 0.5 and 1 : 1, and wherein the quantity of nitrogen in the nitrogen source is 0.005 - 0.1 kg N per m³ H₂, preferably 0.01 - 0.05 kg N per m³ H₂.

14. A method according to any of claims 12 - 13, wherein the hydrogen oxidizing bacteria are knallgas bacteria from the genus of *Ralstonia, Proteobacteria, Aquificales, Actinobacteria, Firmicutes. Sulfuricurvum, Alcaligenes, Cupriavidus, Thermomonas, Chryseobacterium, Ancylobacter, Hydrogenophaga, Xanthobacter, Bdellovibrio, Falvobacteria, Sphingobacteria, Ideonella* or *Streptomyces.*

15. A method according to any of claims 12 - 14, wherein hydrogen is alternatingly provided from a different inlet, and wherein preferably, the alternatingly providing of hydrogen through different inlets is achieved through a cycle in which at no time oxygen is being provided through an inlet where in the preceding step hydrogen has been provided, and in which cycle at no time hydrogen is provided through an inlet where in the preceding step oxygen has been supplied.

16. A method according to claim 15, wherein before and after providing hydrogen through an inlet, that inlet is used to provide a liquid or a gas different from hydrogen or oxygen, and wherein before and after providing oxygen through an inlet, that inlet is used to provide a liquid or a gas different from hydrogen or oxygen.

17. A method according to claim 16, wherein the liquid is water, or a solution comprising ammonium ions, nitrite ions, nitrate ions, and/or amino acids, wherein preferably, the liquid has a temperature of 40 - 80 °C.

## Patentansprüche

1. Bioreaktor für aerobe hydrogenotrophe Fermentation, umfassend einen oberen Teil, umfassend ein Kopfende, einen unteren Teil, umfassend einen Boden, einen Zwischenteil, umfassend eine umlaufende Seitenfläche, und eine Schleife, angepasst, um Fließen einer Fermentationsbrühe von einem Austrittspunkt in dem Bodenteil zu einem Eintrittspunkt in dem oberen Teil zu ermöglichen, wobei der Bioreaktor einen Innendurchmesser (D) und eine Höhe (h) hat, definiert durch den vertikalen Abstand zwischen dem Kopfende und dem Boden, und wobei der Bioreaktor **gekennzeichnet ist durch**
- eine Pumpe, angepasst, um einen Kreislaufstrom der Fermentationsbrühe von dem oberen Teil zu dem unteren Teil des Reaktors und durch die Schleife von dem Austrittspunkt in dem unteren Teil zu dem Eintrittspunkt in dem oberen Teil bereitzustellen;
- einen ersten Gaseinlass, angepasst, um Wasserstoffblasen bereitzustellen;
- einen zweiten Gaseinlass, angeordnet auf derselben Höhe oder unterhalb des ersten Gaseinlasses, angepasst, um Sauerstoff bereitzustellen;
wobei der Zwischenteil als der Teil zwischen dem ersten Gaseinlass und dem Eintrittspunkt in dem Oberteil definiert ist, und wobei die umlaufende Seitenfläche des Zwischenteils einen konischen Abschnitt umfasst, definiert durch einen Innendurchmesser (D), der mit der Höhe (h) variiert, so dass eine geringere Höhe in einem größeren Durchmesser resultiert.

2. Bioreaktor nach Anspruch 1, wobei die Wasserstoffblasen eine durchschnittliche Größe von 20 - 300 µm haben.

3. Bioreaktor nach Anspruch 1 oder 2, wobei der erste Gaseinlass konfiguriert ist, um einen kontinuierlichen Strom von Wasserstoffblasen bereitzustellen, wobei der Strom einen horizontalen Querschnittsoberflächenbereich hat, der wenigstens 15 % des horizontalen Querschnittsoberflächenbereichs der umlaufenden Seitenfläche auf der Höhe, auf der der Strom aus dem ersten Gaseinlass austritt, ist.

4. Bioreaktor nach einem der Ansprüche 1 - 3, wobei die umlaufende Seitenfläche des konischen Abschnitts des Zwischenteils auf wenigstens einer Seite durch ein Seitenansichts-Querschnittsprofil definiert ist, das linear, konkav oder konvex ist.

5. Bioreaktor nach einem der Ansprüche 1 - 4, wobei der Innendurchmesser des konischen Abschnitts mit der Höhe, definiert durch eine lineare Funktion von dem Typ D = a • h+b variiert, wobei a <0 und b eine positive Zahl ist, oder durch eine Exponentialfunktion von dem Typ D = m • n^{h} +c, wobei m und c positive Zahlen sind, und wobei n zwischen 0 und 1.

6. Bioreaktor nach einem der Ansprüche 1 - 5, ferner umfassend
(a) einen oder mehrere zusätzliche Einlässe, angepasst, um eine Kohlenstoffquelle und/oder eine Stickstoffquelle in den Reaktor einzubringen; und/oder
(b) einen dritten Gaseinlass, angepasst, um eine Kohlenstoffquelle bereitzustellen, wobei die Kohlenstoffquelle Kohlenmonoxid, Kohlendioxid, oder eine Mischung aus Kohlenmonoxid und Kohlendioxid ist; und/oder
(c) einen vierten Gaseinlass, angepasst, um als Stickstoffquelle Ammoniakgas bereitzustellen und/oder
(d) einen Flüssigkeitseinlass, angepasst, um als Stickstoffquelle eine Lösung, umfassend Ammoniumionen, Nitritionen, Nitrationen, und/oder Aminosäuren bereitzustellen.

7. Bioreaktor nach einem der Ansprüche 1 - 6, konfiguriert zur Fermentation von wasserstoffoxidierenden Bakterien.

8. Bioreaktor nach einem der Ansprüche 1 - 7, wobei die umlaufende Seitenfläche einen horizontale Querschnittsoberflächenbereich hat, der auf jeder Höhe symmetrisch ist.

9. Bioreaktor nach einem der Ansprüche 1 - 8, umfassend wenigstens zwei Einlässe, wobei die Einlässe angepasst sind, um entweder Gasblasen von 20 - 300 µm, oder Flüssigkeit bereitzustellen, so dass die Wasserstoffblasen abwechselnd von einem anderen Einlass bereitgestellt werden.

10. Bioreaktor nach einem der Ansprüche 1 - 9, wobei sowohl der erste Gaseinlass als auch der zweite Gaseinlass angepasst sind, um entweder Wasserstoffblasen oder Sauerstoffblasen bereitzustellen, so dass der zweite Gaseinlass Sauerstoffblasen bereitstellt, wenn der erste Gaseinlass Wasserstoffblasen bereitstellt, und der zweite Gaseinlass Wasserstoffblasen bereitstellt, wenn der erste Gaseinlass Sauerstoffblasen bereitstellt, wobei der erste und der zweite Gaseinlass mit einer Sauerstoffgaszufuhr und einer Wasserstoffgaszufuhr schaltbar verbunden sind, so dass der erste Gaseinlass und der zweite Gaseinlass abwechselnd Wasserstoffgasblasen und Sauerstoffgasblasen bereitstellen.

11. Bioreaktor nach einem der Ansprüche 1 - 10, wobei der erste Gaseinlass angepasst ist, um entweder Wasserstoffblasen oder eine Flüssigkeit bereitzustellen, wobei der erste Gaseinlass mit einer Wasserstoffgaszufuhr und einer Flüssigkeitszufuhr schaltbar verbunden ist, so dass der erste Gaseinlass abwechselnd Wasserstoffgasblasen und Flüssigkeit bereitstellt, wobei vorzugsweise die Flüssigkeit Wasser ist, wobei das Wasser optional Wasserstoffperoxid und/oder Säure umfasst, wobei das Wasser vorzugsweise eine Temperatur von 40 - 80 °C hat, oder wobei die Flüssigkeit eine wässrige Lösung ist, umfassend Ammoniumionen, Nitritionen, Nitrationen und/oder Aminosäuren.

12. Verfahren für die Herstellung von Biomasse, umfassend
- Bereitstellen eines Bioreaktors nach einem der Ansprüche 1 - 11 mit ausreichend Wasser, ausreichend Kohlenstoffquelle, ausreichend Sauerstoffquelle, ausreichend Stickstoffquelle, und ausreichend Nährstoffen, um eine Fermentation aufrechtzuerhalten, und mit einem Inokulum, umfassend wasserstoffoxidierende Bakterien, und Bereitstellen von Wasserstoffblasen mit einer durchschnittlichen Größe von 20 - 300 µm, um eine Fermentationsbrühe zu erhalten;
- Betreiben der Pumpe, um die Fermentationsbrühe von dem oberen Teil durch den Zwischenteil zum unteren Teil des Reaktors, und durch die Schleife von dem unteren Teil zu dem oberen Teil zu zirkulieren, so dass sich die Wasserstoffblasen im Wesentlichen vollständig in der Fermentationsbrühe lösen;
- Fortsetzen von Fermentation, bis eine extrahierbare Menge von Biomasse erzeugt wurde;
- Isolieren von Protein aus der Fermentationsbrühe.

13. Verfahren nach Anspruch 12, wobei die Kohlenstoffquelle gasförmiges Kohlendioxid oder Kohlenmonoxid ist, die Sauerstoffquelle gasförmiger Sauerstoff ist, und die Stickstoffquelle entweder gasförmiger Ammoniak oder eine Lösung, umfassend Ammoniumionen, Nitritionen, Nitrationen und/oder Aminosäuren, wobei vorzugsweise das Volumenverhältnis zwischen Wasserstoff und Sauerstoff zwischen 0,5 : 1 und 10 : 1, vorzugsweise zwischen 1: 1 und 5 : 1 ist und wobei das Verhältnis zwischen Sauerstoff und Kohlendioxid zwischen 1: 0,1 und 1 : 1,5, vorzugsweise zwischen 1: 0,5 und 1 : 1 ist, und wobei die Stickstoffmenge in der Stickstoffquelle 0,005 - 0,1 kg N pro m³ H₂, vorzugsweise 0,01 - 0,05 kg N pro m³ H₂ ist.

14. Verfahren nach einem der Ansprüche 12 - 13, wobei die wasserstoffoxidierenden Bakterien Knallgasbakterien aus der Gattung von *Ralstonia, Proteobacteria, Aquificales, Actinobacteria, Firmicutes, Sulfuricurvum, Alcaligenes, Cupriavidus, Thermomonas, Chryseobacterium, Ancylobacter, Hydrogenophaga, Xanthobacter, Bdellovibrio, Falvobacteria, Sphingobacteria, Ideonella* oder *Streptomyces* sind.

15. Verfahren nach einem der Ansprüche 12 - 14, wobei Wasserstoff abwechselnd von einem anderen Einlass bereitgestellt wird, und wobei vorzugsweise das abwechselnde Bereitstellen von Wasserstoff durch verschiedene Einlässe durch einen Zyklus erreicht wird, in dem zu keinem Zeitpunkt Sauerstoff durch einen Einlass bereitgestellt wird, wo in dem vorhergehenden Schritt Wasserstoff bereitgestellt wurde, und in dem Zyklus zu keinem Zeitpunkt Wasserstoff durch einen Einlass bereitgestellt wird, wo in dem vorhergehenden Schritt Sauerstoff zugeführt wurde.

16. Verfahren nach Anspruch 15, wobei vor und nach Bereitstellen von Wasserstoff durch einen Einlass, dieser Einlass verwendet wird, um eine Flüssigkeit oder ein Gas, verschieden von Wasserstoff oder Sauerstoff bereitzustellen, und wobei vor und nach dem Bereitstellen von Sauerstoff durch einen Einlass, dieser Einlass verwendet wird, um eine Flüssigkeit oder ein Gas, verschieden von Wasserstoff oder Sauerstoff, bereitzustellen.

17. Verfahren nach Anspruch 16, wobei die Flüssigkeit Wasser oder eine Lösung umfassend Ammoniumionen, Nitritionen, Nitrationen, und/oder Aminosäuren ist, wobei vorzugsweise die Flüssigkeit eine Temperatur von 40 - 80 °C hat.

## Revendications

1. Bioréacteur pour la fermentation hydrogénotrophe aérobie, comprenant une partie supérieure comprenant un haut, une partie inférieure comprenant un fond, une partie intermédiaire comprenant une face latérale circonférentielle et une boucle adaptée pour permettre l'écoulement d'un bouillon de fermentation à partir d'un point de sortie dans le partie inférieure à un point d'entrée dans la partie supérieure, lequel bioréacteur a un diamètre intérieur (D) et une hauteur (h) définis par la distance verticale entre le haut et le bas, et lequel bioréacteur est **caractérisé par**
- une pompe adaptée pour fournir un écoulement circulaire du bouillon de fermentation de la partie supérieure à travers la partie intermédiaire vers la partie inférieure du réacteur, et à travers la boucle du point de sortie dans la partie inférieure au point d'entrée dans la partie supérieure ;
- une première entrée de gaz, adaptée pour fournir des bulles d'hydrogène ;
- une seconde entrée de gaz située à la même hauteur ou en dessous de la première entrée de gaz, adaptée pour fournir de l'oxygène ;
dans lequel la partie intermédiaire est définie comme la partie entre la première entrée de gaz et le point d'entrée dans la partie supérieure, et dans laquelle la face latérale circonférentielle de la partie intermédiaire comprend une partie conique définie par un diamètre intérieur (D) qui varie avec la hauteur (h) de telle sorte qu'une hauteur inférieure entraîne un diamètre plus grand.

2. Bioréacteur selon la revendication 1, dans lequel les bulles d'hydrogène ont une taille moyenne de 20 à 300 µm.

3. Bioréacteur selon la revendication 1 ou 2, dans lequel la première entrée de gaz est configurée pour fournir un flux continu de bulles d'hydrogène, le flux ayant une surface transversale horizontale qui représente au moins 15% de la surface transversale horizontale de la face latérale circonférentielle à la hauteur où le flux émerge de la première entrée de gaz.

4. Bioréacteur selon l'une quelconque des revendications 1 à 3, dans lequel la face latérale circonférentielle de la partie conique de la partie intermédiaire est définie sur au moins un côté par un profil en coupe latérale qui est linéaire, concave ou convexe.

5. Bioréacteur selon l'une quelconque des revendications 1 à 4, dans lequel le diamètre intérieur de la partie conique varie avec la hauteur définie par une fonction linéaire du type D = a ^{∗} h + b dans laquelle a <0 et b est un nombre positif, ou par une fonction exponentielle du type D = m ^{∗} n^{h} + c, où m et c sont des nombres positifs, et où n est compris entre 0 et 1.

6. Bioréacteur selon l'une quelconque des revendications 1 à 5, comprenant en outre
(a) une ou plusieurs entrées supplémentaires, adaptées pour introduire une source de carbone et/ou une source d'azote dans le réacteur ; et/ou
(b) une troisième entrée de gaz adaptée pour fournir une source de carbone, la source de carbone étant du monoxyde de carbone, du dioxyde de carbone ou un mélange de monoxyde de carbone et de dioxyde de carbone ; et/ou
(c) une quatrième entrée de gaz, adaptée pour fournir comme source d'azote, du gaz ammoniac, et/ou
(d) une entrée de liquide, adaptée pour fournir comme source d'azote une solution comprenant des ions ammonium, des ions nitrite, des ions nitrate, et/ou acides aminés.

7. Bioréacteur selon l'une quelconque des revendications 1 à 6, configuré pour la fermentation de bactéries oxydant l'hydrogène.

8. Bioréacteur selon l'une quelconque des revendications 1 à 7, dans lequel la face latérale circonférentielle a une surface transversale horizontale qui est symétrique à n'importe quelle hauteur.

9. Bioréacteur selon l'une quelconque des revendications 1 à 8, comprenant au moins deux entrées, lesquelles entrées sont adaptées pour fournir soit des bulles de gaz de 20 à 300 µm, soit du liquide, de sorte que les bulles d'hydrogène sont alternativement fournies à partir d'une entrée différente.

10. Bioréacteur selon l'une quelconque des revendications 1 à 9, dans lequel à la fois la première entrée de gaz et la seconde entrée de gaz sont adaptées pour fournir soit des bulles d'hydrogène soit des bulles d'oxygène, de sorte que la deuxième entrée de gaz fournit des bulles d'oxygène lorsque la première entrée de gaz fournit des bulles d'hydrogène, et la deuxième entrée de gaz fournit des bulles d'hydrogène lorsque la première entrée de gaz fournit des bulles d'oxygène, la première et la deuxième entrée de gaz étant connectées de manière commutable à une alimentation en oxygène gazeux et à une alimentation en hydrogène gazeux, de sorte que la première entrée de gaz et la seconde L'entrée de gaz fournit alternativement des bulles d'hydrogène gazeux et des bulles d'oxygène gazeux.

11. Bioréacteur selon l'une quelconque des revendications 1 à 10, dans lequel la première entrée de gaz est adaptée pour fournir soit des bulles d'hydrogène soit un liquide, dans lequel la première entrée de gaz est connectée de manière commutable à une alimentation en hydrogène gazeux et à une alimentation en liquide, de telle sorte que le la première entrée de gaz fournit alternativement des bulles d'hydrogène gazeux et du liquide, dans lequel de préférence le liquide est de l'eau, laquelle eau comprend facultativement du peroxyde d'hydrogène et/ou de l'acide, laquelle eau a de préférence une température de 40 à 80°C, ou dans laquelle le liquide est une solution aqueuse comprenant des ions ammonium, des ions nitrite, des ions nitrate et/ou des acides aminés.

12. Procédé pour la production de biomasse, comprenant
- la fourniture d'un bioréacteur selon l'une quelconque des revendications 1 à 11 avec suffisamment d'eau, une source de carbone suffisante, une source d'oxygène suffisante, une source d'azote suffisante et des nutriments suffisants pour soutenir une fermentation, et avec un inoculum comprenant des bactéries oxydant l'hydrogène et fournissant des bulles d'hydrogène ayant une taille moyenne de 20 à 300 µm, pour obtenir un bouillon de fermentation ;
- faire fonctionner la pompe pour faire circuler le bouillon de fermentation de la partie supérieure à travers la partie intermédiaire vers la partie inférieure du réacteur, et à travers la boucle de la partie inférieure à la partie supérieure, de sorte que les bulles d'hydrogène se dissolvent essentiellement complètement dans le bouillon de fermentation ;
- poursuivre la fermentation jusqu'à ce qu'une quantité extractible de biomasse soit générée ;
- isoler les protéines du bouillon de fermentation.

13. Procédé selon la revendication 12, dans lequel la source de carbone est du dioxyde de carbone gazeux ou du monoxyde de carbone, la source d'oxygène est de l'oxygène gazeux et la source d'azote est soit de l'ammoniac gazeux, soit une solution comprenant des ions ammonium, des ions nitrite, des ions nitrate, et/ou des acides aminés, dans lesquels de préférence, le rapport volumétrique entre l'hydrogène et l'oxygène est compris entre 0,5 : 1 et 10 : 1, de préférence entre 1 : 1 et 5 : 1, et dans lequel le rapport entre l'oxygène et le dioxyde de carbone est compris entre 1 : 0,1 et 1 : 1,5, de préférence entre 1 : 0,5 et 1 : 1, et dans lequel la quantité d'azote dans la source d'azote est de 0,005 - 0,1 kg N par m³ H₂, de préférence 0,01 - 0,05 kg N par m³ H₂.

14. Procédé selon l'une quelconque des revendications 12 à 13, dans lequel les bactéries oxydant l'hydrogène sont des bactéries knallgas du genre *Ralstonia, Proteobacteria, Aquificales, Actinobacteria, Firmicutes. Sulfuricurvum, Alcaligenes, Cupriavidus, Thermomonas, Chryseobacterium, Ancylobacter, Hydrogenophaga, Xanthobacter, Bdellovibrio, Falvobacteria, Sphingobacteria, Ideonella ou Streptomyces.*

15. Procédé selon l'une quelconque des revendications 12 à 14, dans lequel l'hydrogène est fourni en alternance à partir d'une entrée différente, et dans lequel de préférence, la fourniture en alternance d'hydrogène à travers différentes entrées est réalisée par un cycle dans lequel à aucun moment l'oxygène n'est fourni à travers une entrée où à l'étape précédente de l'hydrogène a été fourni, et dans quel cycle à aucun moment l'hydrogène n'est fourni à travers une entrée où à l'étape précédente l'oxygène a été fourni.

16. Procédé selon la revendication 15, dans lequel avant et après la fourniture d'hydrogène à travers une entrée, cette entrée est utilisée pour fournir un liquide ou un gaz différent de l'hydrogène ou de l'oxygène, et dans lequel avant et après la fourniture d'oxygène à travers une entrée, cette entrée est utilisée pour fournir un liquide ou un gaz différent de l'hydrogène ou de l'oxygène.

17. Procédé selon la revendication 16, dans lequel le liquide est de l'eau ou une solution comprenant des ions ammonium, des ions nitrite, des ions nitrate et/ou des acides aminés, dans lequel de préférence, le liquide a une température de 40 à 80°C.
